# EUROPEAN PATENT APPLICATION

(11) **EP 4 202 058 A1**
(43) Date of publication of application: **28.06.2023**
(21) Application number: 21951111.0
(22) Date of filing: 18.11.2021
(51) Int. Cl.: C12Q 1/6869, C12N 15/11, C40B 50/06

(54) **LIBRARY CONSTRUCTION ELEMENT COMPATIBLE WITH DOUBLE SEQUENCING PLATFORMS, KIT, AND LIBRARY CONSTRUCTION METHOD**

(30) Priority: 09.11.2021 CN 202111322946
(71) Applicant: Nanodigmbio (Nanjing) Biotechnology Co., Ltd, Jiangsu 210000 (CN)
(72) Inventor: WANG, Biao, Nanjing, Jiangsu 210000 (CN); HU, Yugang, Nanjing, Jiangsu 210000 (CN); WU, Qiang, Nanjing, Jiangsu 210000 (CN)
(74) Representative: Casalonga
(86) International application number: PCT/CN2021/131508
(87) International publication number: WO 2023/082305

(57) **Abstract**

The present application provides a library construction element, a kit and a library construction method compatible with double sequencing platforms. The library construction method comprises: performing library construction on target samples using primers or adaptors with a 5' phosphorylation modification to obtain a linear amplification library with a 5' phosphorylation modification, that is, a linear library suitable for Illumina sequencing platform; or further cyclizing the linear amplification library with a 5' phosphorylation modification to obtain a cyclization library suitable for the MGI sequencing platform. By the phosphorylation modification on the 5' end of the Illumina full-length adaptor or the 5' end band of the library amplification primer, it is convenient to obtain a linear library suitable for the Illumina sequencing platform, and at the same time, the cyclization library suitable for the MGI sequencing platform can be obtained by direct cyclization using the 5'end phosphorylation modification. Thus, the problem of low compatibility of the existing library construction methods on the two platforms is solved.

## Description

### Technical Field

The present application relates to the field of next generation sequencing library construction, in particular to a library construction method, a kit and a library construction element compatible with double sequencing platforms.

### Background

Next generation sequencing is to obtain sequence information by measuring nucleic acid sequence. At present, the mainstream second-generation sequencers are sequencers from Illumina, MGI and Life, of which the Illumina sequencer dominants, next is the MGI sequencer. At the same time, these two sequencers have the same sequencing principle, which achieves the reading of the nucleic acid sequence by marking different fluorescent signals for mononucleotides in the synthesis process through Sequencing by Synthesis. While the Life sequencer implements detection through the release of electronic signals in the synthesis process.

Since the sequencing by Illumina and MGI depends on fluorescent labeled mononucleotides, with the development of technology, the Illumina sequencer has developed three fluorescent channels and corresponding photographic modes, that is, four-color fluorescence four-channel mode, three-color fluorescence two-channel mode and four-color single-channel mode. The MGI sequencer also has four-color fluorescence four-channel mode and three-color fluorescence two-channel mode. Therefore, these sequencing modes all need to detect fluorescent signals. Since fluorescent signals overlap with each other, filters are required to avoid mutual interference during detection. Another solution is to schedule with base equalization during mixed testing as much as possible. Likewise, it is also needed to consider the balance problem for the Index sequences. If the balance problem is well considered in the design of Index, it is unnecessary to consider the loading problem when arranging various channels.

At the same time, since Illumina's loading mode uses linear amplification to perform computer sequencing, while MGI's loading mode uses cyclized library for sequencing. In this way, if it is desired to load and sequence the Illumina's library, it is needed to be processed by a separate workflow before cyclization, and the current processing method is to use PCR amplification. Although this method can solve the loading problem of the Illumina library on the MGI sequencing platform, it increases the operation workflow and the redundancy of the sequencing data.

Therefore, there is a need to provide a simple and effective library construction solution compatible with the two sequencing platforms in the market at present.

### Summary

The main object of the present application is to provide a library construction element, kit and library construction method compatible with double sequencing platforms, so as to solve the problem that the library construction solutions in the prior art has low compatibility on the two sequencing platforms.

In order to achieve the above object, according to one aspect of the present application, a library construction method compatible with double sequencing platforms is provided, the library construction method comprises: constructing a library for a target sample with primers having a 5' phosphorylation modification or adaptors with a 5' phosphorylation modification to obtain a linear amplification library with a 5' phosphorylation modification, and the linear amplification library with a 5' phosphorylation modification being a linear library suitable for the Illumina sequencing platform; or further cyclizing the linear amplification library with a 5' phosphorylation modification to obtain a cyclization library suitable for the MGI sequencing platform; wherein, the primers having a 5' phosphorylation modification comprises a P5 truncated amplification primer as shown in SEQ ID NO: 1 and a P7 truncated amplification primer as shown in SEQ ID NO: 2; and the adaptors having a 5' phosphorylation modification comprises a P5 full-length adaptor as shown in SEQ ID NO: 3 and a P7 full-length adaptor as shown in SEQ ID NO: 4; wherein, SEQ ID NO: 1: /5Phos/AATGATACGGCGACCACCGAGATCTACACNNNNNNNNNNACACTCTTTCCCTACAC GAC, 10 N(s) represent a P5 end index sequence; SEQ ID NO: 2: CAAGCAGAAGACGGCATACGAGATNNNNNNNNNNGTGACTGGAGTTCAGACGTGT, 10 N(s) represent a P7 end index sequence; SEQ ID NO: 3: /5Phos/AATGATACGGCGACCACCGAGATCTACACNNNNNNNNNNACACTCTTTCCCTACAC GACGCTCTTCCGATC*T, 10 N(s) represent a P5 end index sequence, * represents a phosphorthioate modification; SEQ ID NO: 4: /5Phos/GATCGGAAGAGCACACGTCTGAACTCCAGTCACNNNNNNNNNNATCTCGTATGCCG TCTTCTGCTTG, 10 N(s) represent a P7 end index sequence; wherein, each 1 bp upstream and downstream of index sequences comprising the P5 end index sequence or the P7 end index sequence contains at least three editing distances.

Further, the P5 end index sequence is selected from any one of Table 1-1, and the P7 end index sequence is selected from any one of Table 1-2.

Further, there are multiple target samples, multiple P5 end index sequences corresponding to the multiple target samples are selected from any group of the 4-base balanced index sequences in Table 1-1, and multiple P7 end index sequences corresponding to the multiple target samples are selected from any group of the 4-base balanced index sequences in Table 1-2, the 4-base balanced index sequence refers to a group of four index sequences balanced, that is, each base of A, T, G and C appears once at each position from position 1 to position 10 of the four index sequences.

Further, the primers having a 5' phosphorylation modification is used to perform the library construction on the target sample to obtain the linear amplification library with a 5'phosphorylation modification, comprising: applying the truncated adaptors as shown in SEQ ID NO: 7 and SEQ ID NO: 8 to carry out an adaptor ligation on the fragments originated from the target sample to obtain the fragments with adaptors; applying the primers having a 5' phosphorylation modification as shown in SEQ ID NO: 1 and SEQ ID NO: 2 to amplify the fragments with adaptors to obtain the linear amplification library with the 5' phosphorylation modification; wherein, SEQ ID NO: 7: ACACTCTTTCCCTACACGACGCTCTTCCGATC*T, * represents a phosphorthioate modification; SEQ ID NO: 8: /5Phos/GATCGGAAGAGCACACGTCTGAACTCCAGTCAC.

Further, constructing a library for an target sample with adaptors having a 5' phosphorylation modification to obtain the linear amplification library with a 5'phosphorylation modification, comprising: applying the full-length adaptor as shown in SEQ ID NO: 3 and SEQ ID NO: 4 to carry out an adaptor ligation on the fragments originated from the target sample to obtain the libraries with adaptors; amplifying the libraries with adaptors with the library amplification primers as shown in SEQ ID NO: 5 and SEQ ID NO: 6 to obtain the linear amplification library with a 5'phosphorylation modification; wherein, SEQ ID NO: 5 : /5Phos/AATGATACGGCGACCACCGAGAT; SEQ ID NO: 6 : CAAGCAGAAGACGGCATACGA.

Further, before cyclization, the library construction method further comprises the step of targeted capturing the linear amplification library; preferably, amplifying the captured library after the targeted capturing with a library amplification primers having a 5'phosphorylation modification to obtain a linear amplification captured library, cyclizing the linear amplification captured library to obtain the cyclization library suitable for the MGI sequencing platform; preferably, the library amplification primers having a 5'phosphorylation modification comprises a P5 phosphorylation primer as shown in SEQ ID NO: 5 and a P7 primer as shown in SEQ ID NO: 6.

According to a second aspect of the present application, a library construction kit compatible with double sequencing platforms is provided, the library construction kit comprises any of the following combinations: 1) combination 1: a P5 truncated amplification primer as shown in SEQ ID NO: 1 and a P7 truncated amplification primer as shown in SEQ ID NO: 2, wherein, SEQ ID NO: 1: /5Phos/AATGATACGGCGACCACCGAGATCTACACNNNNNNNNNNACACTCTTTCCCTACAC GAC, 10 N(s) represent a P5 end index sequence; SEQ ID NO: 2: CAAGCAGAAGACGGCATACGAGATNNNNNNNNNNGTGACTGGAGTTCAGACGTGT, 10 N(s) represent a P7 end index sequence; 2) combination 2: a P5 full-length adaptor as shown in SEQ ID NO: 3 and a P7 full-length adaptor as shown in SEQ ID NO: 4, wherein, SEQ ID NO: 3: /5Phos/AATGATACGGCGACCACCGAGATCTACACNNNNNNNNNNACACTCTTTCCCTACAC GACGCTCTTCCGATC*T, 10 N(s) represent a P5 end index sequence, * represents the phosphorthioate modification; SEQ ID NO: 4: /5Phos/GATCGGAAGAGCACACGTCTGAACTCCAGTCACNNNNNNNNNNATCTCGTATGCCG TCTTCTGCTTG, 10 N(s) represent a P7 end index sequence; wherein, each 1 bp upstream and downstream of index sequences comprising the P5 end index sequence or the P7 end index sequence contains at least three editing distances.

Further, the P5 end index sequence is selected from any one of Table 1-1, and the P7 end index sequence is selected from any one of Table 1-2.

Further, the library construction kit comprises 412 P5 end index sequences and 432 P7 end index sequences, the P5 end index sequences are as shown in Table 1-1, and the P7 end index sequences are as shown in Table 1-2, wherein, the P5 end index sequence and/or the P7 end index sequence are used in a coordination way of a group of 4-base balanced index sequences.

Further, the library construction kit further comprises library amplification primers as shown in SEQ ID NO: 5 and SEQ ID NO: 6, and/or truncated adaptors as shown in SEQ ID NO: 7 and 8.

According to a third aspect of the present application, a library construction element compatible with double sequencing platforms is provided, the library construction element is selected from any of the following combinations: 1) combination 1: a P5 truncated amplification primer as shown in SEQ ID NO: 1 and a P7 truncated amplification primer as shown in SEQ ID NO: 2, wherein, SEQ ID NO: 1: /5Phos/AATGATACGGCGACCACCGAGATCTACACNNNNNNNNNNACACTCTTTCCCTACAC GAC, 10 N(s) represent a P5 end index sequence; SEQ ID NO: 2: CAAGCAGAAGACGGCATACGAGATNNNNNNNNNNGTGACTGGAGTTCAGACGTGT, N(s) represent a P7 end index sequence; 2) combination 2: a P5 full-length adaptor as shown in SEQ ID NO: 3 and a P7 full-length adaptor as shown in SEQ ID NO: 4, wherein, SEQ ID NO: 3: /5Phos/AATGATACGGCGACCACCGAGATCTACACNNNNNNNNNNACACTCTTTCCCTACAC GACGCTCTTCCGATC*T, 10 N(s) represent the P5 end index sequence, * represents a phosphorthioate modification; SEQ ID NO: 4: /5Phos/GATCGGAAGAGCACACGTCTGAACTCCAGTCACNNNNNNNNNNATCTCGTATGCCG TCTTCTGCTTG, 10 N(s) represent a P7 end index sequence; wherein, each 1 bp upstream and downstream of index sequences comprising the P5 end index sequence or the P7 end index sequence contains at least three editing distances.

Further, the P5 end index sequence is selected from any one of Table 1-1, and the P7 end index sequence is selected from any one of Table 1-2.

Further, the library construction element is an amplification primer composition or an adaptor composition, the amplification primer composition comprises a combination of multiple groups of P5 truncated amplification primers and/or multiple groups of P7 truncated amplification primers, each group of the P5 truncated amplification primers comprises a 4-base balanced index sequences selected from any group of Table 1-1, and each group of the P7 truncated amplification primers comprises a 4-base balanced index sequences selected from any group of Table 1-2; the adaptor composition comprises multiple groups of P5 full-length adaptors and/or multiple groups of P7 full-length adaptors, each group of the P5 full-length adaptors comprises a 4-base balanced index sequences selected from any group of Table 1-1, and each group of the P7 full-length adaptors comprises a 4-base balanced index sequences selected from any group of Table 1-2; the 4-base balanced index sequences refers to a group of four index sequences balanced, that is, each base of A T, G and C appear once at each position from position 1 to position 10 of the four index sequence.

Applying the technical solution of the present application, by the phosphorylation modification on the 5' end of the Illumina full-length adaptor (P5 and P7) or the 5' end of the library amplification primer, it is convenient to obtain a linear library suitable for the Illumina sequencing platform, and at the same time, if the MGI sequencing platform is required, the cyclization library suitable for the MGI sequencing platform can be obtained by direct cyclization applying the 5' end phosphorylation modification. Thus, the problem of low compatibility of the library construction methods in the prior art on the two platforms is solved.

### Brief Description of the Drawings

The accompanying drawings, which form a part of this application, are provided to further understand the present application, the illustrative embodiments of the present application and the description thereof are intended to explain the present application and are not intended to limit thereto. In the drawings:
Figure 1 shows the Illumina library can be loaded for sequencing on the MGI sequencing platform through conversion;
Figure 2 shows a compatible library construction solution that a library can be loaded on double platforms;
Figure 3 shows a compatible loading amplification solution amplified after targeted capturing;
Figure 4 shows the proportion of base deletions in the index sequence region;
Figure 5 shows the factors such as direction and terminal bases to be considered when designing the double sequencing platforms compatible indexes;
Figure 6 shows that 10 bases should be considered when designing the index with the P5 end adaptors, and the factors of former and posterior C and A base should also be considered;
Figure 7 shows that 10 bases should be considered when designing the index with the P7 end adaptors, and the factors of former and posterior T and G base should also be considered;
Figure 8 shows the indexes within the P5 end adaptor in the Illuminia patent, when the end base is A, 3 editing distances is changed to 2 editing distances;
Figure 9 shows that there are many 8bp indexes of the IDT version currently used by Illumia, with only two editing distances;
Figure 10 shows that different sequencers have great differences in two-color and four balanced index balancing when sequencing;
Figure 11 shows that the IDT version does not consider the balance;
Figure 12 shows the comparison of library construction outputs between two compatible solutions and Illumina separate solutions;
Figure 13 shows the output of 96 groups of library amplification primer library in embodiment 1 of the present application;
Figure 14 shows the splitting of sequencing data on the double platforms of 96 groups of libraries compatible with double platforms;
Figure 15 shows the lowest and highest base proportion of four indexes balanced in 1-12 combinations;
Figure 16 shows the comparison of data splitting between the present application and 8 groups and 12 groups of mixed chips of IDT version recommended by Illumina.

### Detailed Description of the Embodiments

It should be noted that the Examples in the present application and the features in the Examples may be combined with each other without conflicting. The present application will be described in detail below with reference to the embodiments.

Double ended index adaptors: during the next generation sequencing, each fragment end needs to be ligated to a universal sequencing adaptor. The non-complementary regions of the adaptors each have a variable sequence, which is an index sequence, and is used to split data during sequencing.

Four-base balanced index sequence: the DNA sequence is composed of four bases, namely, A, T, G and C. In order to effectively read during the sequencing process, a group of index sequences are combined to ensure that the proportion of bases at each position of the index sequence is equal.

Compatible double sequencing platform: it means that sequencing on both Illumina sequencing platform and MGI sequencing platform are considered for the library construction adaptor or the amplification primer.

Next generation sequencing (NGS) is an important and widely used Massively Parallel Sequencing (hereinafter referred to as MPS) technology. At present, the suppliers of MPS technology include Illumina, MGI and Ion Torrent, among them, Illumina and MGI sequencers are widely used in the market. Many companies and large research institutions are equipped with these two types of sequencers, i.e. Illumina and MGI sequencers, which have the same sequencing principle and sequencing quality. Therefore, if there is a common library construction solution that can perform undifferentiated loading and sequencing on the two sequencing platforms, it may reduce a lot of trouble and make it easier for relevant personnel to use.

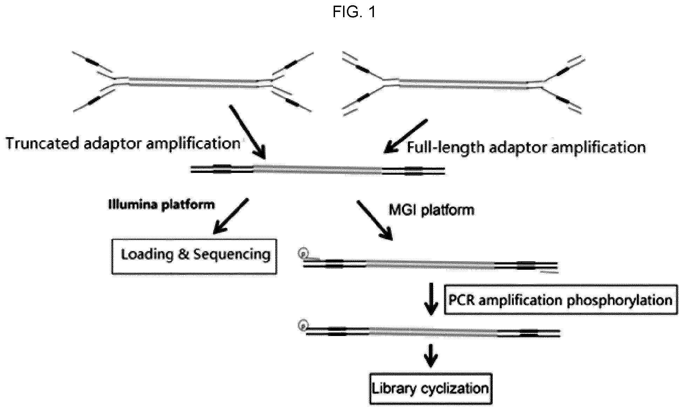

The applicant has developed the library construction solutions for single and dual end indexes of the MGI sequencing platform, as well as Illumina's library construction solutions. The current Illumina library construction solution may only be loaded on the Illumina sequencing platform, and If loaded on the MGI sequencer, the transformation of MGI's App-a (currently, it is one of the ways that Illumina library can be loaded, namely, the transformation protocol of App-a, which specifically uses 3-5 rounds of PCR amplification for terminal phosphorylation, as shown in Figure 1) is needed, at the same time since the design of this index does not consider the base balance of the four indexes, it is difficult to schedule when loading for sequencing. Moreover, for users who have both Illumina and MGI sequencers, it is also troublesome to use two sets of library construction solutions, respectively and a full set of hybrid capture solutions in the later stage. Although MGI currently provides the transformation protocol as shown in Figure 1, which performs transformation and amplification operations on the basis of the established Illumina library, there are two main problems in the workflow of this protocol: (1) increasing the operation workflow, it is necessary to add another transformation process to the established library, which wastes time, manpower and material costs; (2) due to the addition of an amplification (3-5 cycles) in the transformation process, redundancy (Duplications) in the sequencing process is artificially brought about. The original redundancy of the MGI sequencing platform is lower than that of Illumina sequencing platform. On MGISEQ-2000 sequencing platform, if sequencing as per 150 × sequencing depth, the redundancy of whole exons can be controlled at about 2%. The Illumina sequencing platform NovaSeq 6000 has 20% of redundancy from the platform itself. Therefore, through the MGI transformation protocol, the advantage of low redundancy of MGI sequencing platform itself is lost.

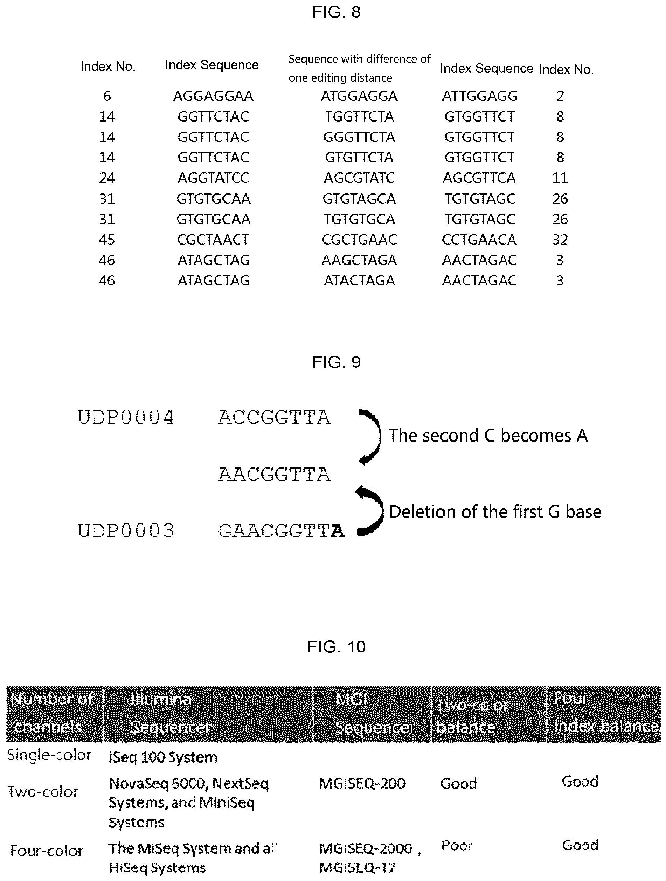

In addition, as shown in Figure 10, since both MGI sequencing platform and Illumina sequencing platform have two-color channel and four-color channel sequencing machines, the library developed on Illumina platform and the publicly available patents for Illumina platform do not attach importance to the base balance of the four-color channel, which is very unfavorable for accurate reading of index sequences.

For this reason, the present application fully considers the problem of two-color and four-color channels, and carries out the absolute balance of four bases in a group of four indexes for the design of index, so that the library construction adaptor of the present application can ensure the sequencing quality of index on both Illumina and MGI sequencing platforms, and also facilitate the operator to easily arrange computer sequencing.

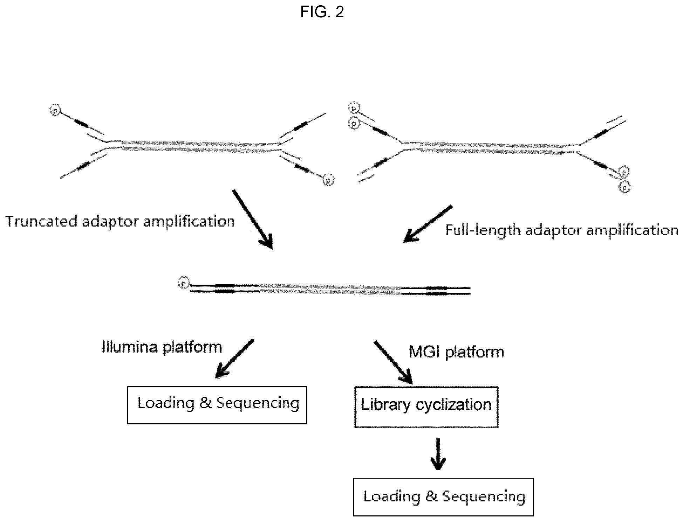

Solution 1 of the present application: the library is constructed through the truncated adaptor ligation, and the dual index sequences are added through amplification. The difference between this solution and the previous library construction solution of the Illumina platform is that the 5' end of the P5 index primer is subjected to phosphorylation modification, so as to ensure that the phosphorylation in the cyclization of the library construction used for the MGI sequencing platform in the later stage can be carried without affecting the amplification, as shown in the left half of Figure 2. The library constructed in this way can be directly loaded on the Illumina sequencing platform or on the MGI sequencing platform after direct cyclization.

Solution 2 of the present application: the adaptor for library construction is a full-length Y-shaped adaptor, and the 5' end of the p5 end of the Y-shaped adaptor has phosphorylation modification. The advantages of such design can achieve PCR free library construction, and sequencing directly performed on the Illumina sequencing platform, or directly cyclized for sequencing on the MGI sequencing platform. At the same time, full-length adaptors can also be directly amplified with p5 and p7 primers having phosphorylation modification and sequenced on Illumina platform or directly cyclized and loaded for sequencing on the MGI sequencing platform.

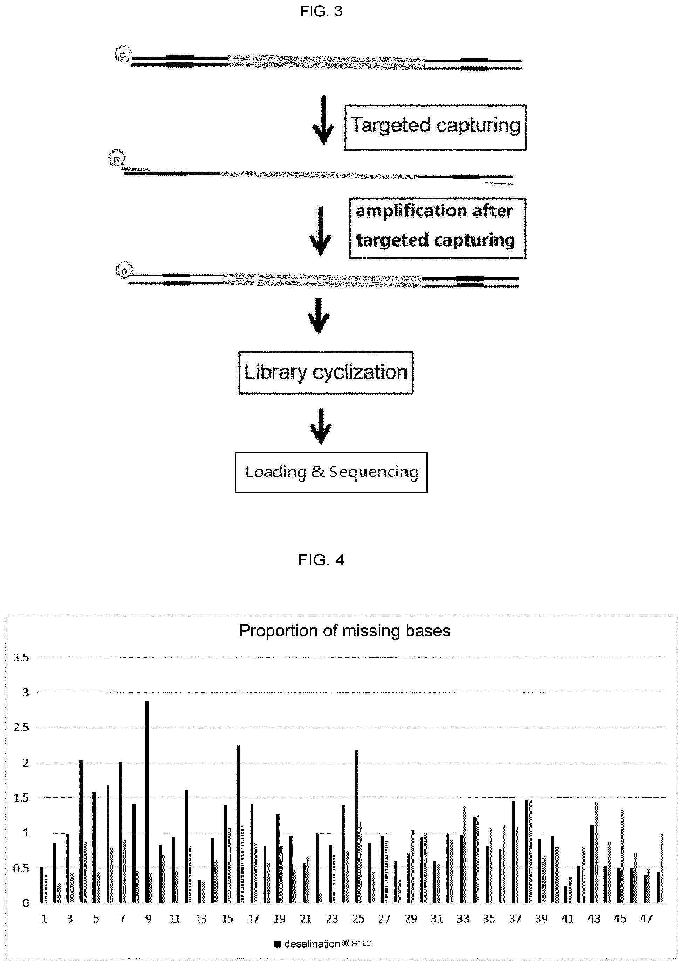

On the basis of the above library construction, the solution of the present application can further be extended to be applied in the targeted capture sequencing. After the targeted capturing, the library is amplified with p5 and p7 having phosphorylation, and the phosphorylation modification only modifies the primer at the p5 end (since the sequencing is orientated, the cyclization on the MGI platform only cyclizes one strand, i.e. only cyclizes the 5' phosphorylated strand at the P5 end). As shown in Figure 3, it ensures that the library amplified after capturing can be sequenced on Illumina sequencing platform or sequenced on the MGI sequencing platform after direct cyclization.

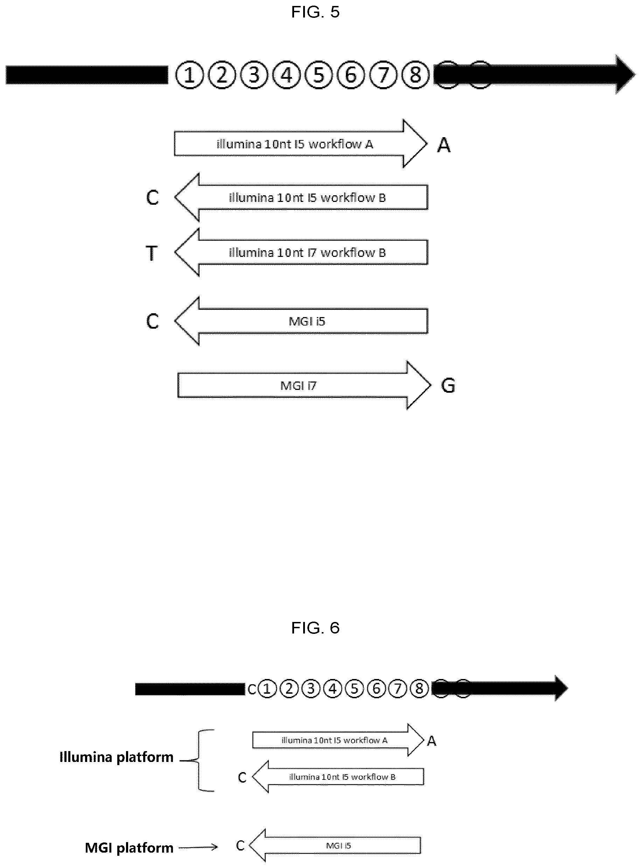

It should be noted that P5/P7 or P5 end/P7 end mentioned in this application refer to P5 and P7 universal sequences of the Illumina sequencing platform. In the actual sequencing process of the present application, it is found that some of the synthetic quality of the index has the phenomenon of base deletion, and it is speculated that there is a certain probability and a certain proportion of base deletion in the process of primer or adaptor synthesis. As shown in Figure 4, by analyzing the data of sequencing the index part, it is found that about 0.2% - 2.8% of the single base deletions exists in the desalted and HPLC purified sequences synthesized by IDT. The deletions can be improved by HPLC purification but cannot be eliminated fundamentally. At the same time, considering that the next base will be sequenced step by step due to the loss of index during the sequencing process, the first base after the index should also be considered when optimizing the index sequence, as shown in Figure 5. Since all sequencing directions of Illumina and MGI are considered, the first base in the former and posterior directions of the P5 end index and the P7 end index should be considered.

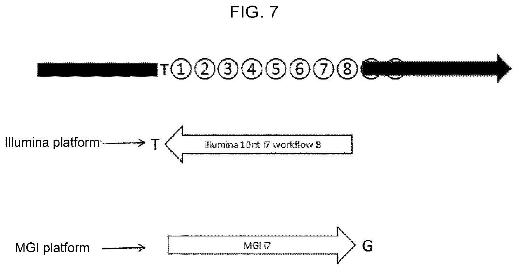

As shown in Figure 6, the index sequence at P5 end has both forward and reverse sequencing for different models of sequencers on the Illumina sequencing platform, but reverse sequencing for sequencers on the MGI platform. Therefore, for the P5 end, the C base at the anterior end of the index (i.e. the next base of the index sequenced in the forward direction) and the A base at the posterior end (i.e. the next base of the index sequenced in the reverse direction) should be considered. Similarly, as shown in Figure 7, the index sequence at P7 end is the reverse sequencing of the index sequence on the Illumina sequencing platform, it is necessary to consider the base T at the anterior end, the MGI platform reads the index in the forward direction, and it is necessary to consider the base G at the posterior end. Due to the need to consider the difference changes before different indexes caused by missing complements and sequencing errors, this problem has not been mentioned in the previously published articles and published patent literatures.

For example, in Illumina's patent PCT/US2018059255, some indexes that meet the three editing distances will become only two editing distances, as shown in Figure 8 (the editing distance in Figure 8 represents the number of different bits corresponding to two words of the same length, and d (x, y) represents the editing distance between two words x and y, exclusive-or operation is performed on two character strings, and the number of numbers whose result is 1 is counted, which is the editing distance, the smaller the distance, the higher the surface similarity). When only one terminal A base at P5 end is considered, the editing distance of 12 indexes in 64 8-bp sequences is changed from three to two. If another direction is considered, more index sequences will not conform to the rule of three editing distances. When designing an index, it should ensure that there are at least three editing distances, so that when one error exists during the analysis, the correct data splitting results can still be found depending on the differences between the other two. If there are only two editing distances, the data cannot be split correctly if any one link is wrong.

Similarly, in the dual index (for example, PMID: 23793624) published by the existing articles, 10 bp index can be calculated 7198 index sequences with 3 editing distances. Considering the 10bp sequence and the first and last bases of the adaptor close to the index, only more than 1000 indexes have strict three editing distances, and it is also needed to consider a group of four bases, and the upstream and downstream bases of P5 and P7 end indexes are considered, respectively, 412 and 432 are suitable for each, as shown in Table 1-1 and Table 1-2.

Likewise, the number of index sequences of 8 bp index with 3 editing distances will also decrease correspondingly. For example, 384 of 8 bp index sequences designed by IDT officially recommended by Illumina sequencing platform have the same problem. As shown in Figure 9, the P5 end index 4 (i.e. UDP0004) of IDT version will become the middle sequence when one base is mutated. As long as the first G base of the index 3 (i.e. UDP0003) sequence is missing, the next A will naturally advance to the anterior sequence and become the middle sequence during sequencing. Therefore, each former and posterior base of the index and the base deletion or mutation are considered together to design the index, which is not mentioned in the previously published articles and the currently published patents, and the currently published index sequences do not have strict three editing distances. Therefore, in order to screen enough sequences with strict 3 editing distances, the dual index sequences with a length of 10 bp is selected in this application.

The present application also found that, since Illumina platform and MGI sequencing platform both have two-color channel and four-color channel sequencers, in order to ensure the sequencing quality of the index, in addition to the sequence information of the former and posterior bases of the index, the base balance between the indexes should also be considered. This problem is not considered in the index sequences of Illumina's products and patents. As shown in Figure 10, when it is considered whether the two-color balance (i.e. two channels) or the absolute balance of four indexes (i.e. four channels) will be more conducive to the loading and sequencing of the double sequencing platform, in order to both have a good performance of sequencing data quality on the sequencers of the two-color channel and the four-color channel of Illumina and MGI platforms, the present application selects the absolute balance of four bases of a set of four indexes.

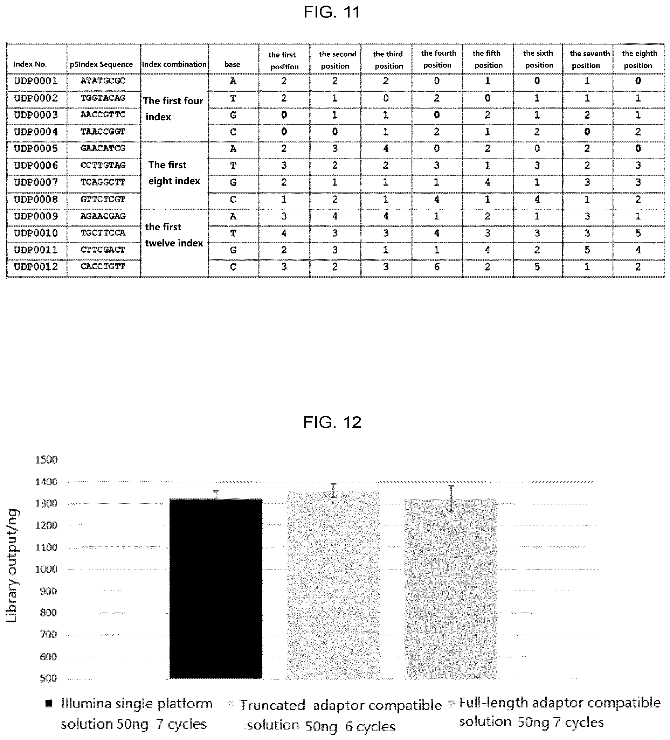

It can be seen from the patents that have been applied and the products that have been sold that the Illumina sequencing platform has not carefully considered this aspect, which also leads to the need to carefully consider the combination of various indexes when scheduling on the Illumina sequencing platform. As shown in Figure 11, the IDT version of P5 end 1-12 index sequence and 4 groups, 8 groups and 12 groups of index base statistics recommended by Illumina sequencing platform show that there are still missing bases in a group of 8 indexes, and the minimum proportion of bases in a group of 12 indexes is 8.3% (for example, the C base at position 7 and the A base at position 8), which is far lower than the MGI sequencer's requirement of no less than 12.5%. The design of absolute balance of bases in the groups of four indexes in the present application has a minimum proportion of bases of greater than 14.28% when the index with an equal amount of greater than or equal to 4 samples is continuously loaded, so it can meet the requirements of various models of the two sequencing platforms. Such index design can not only facilitate the loading schedule, but also improve and ensure the data quality.

It should be noted that the core improvement points of the present application have the following characteristics:
1. The 5' end of amplification primer with index at the truncated p5 end has the phosphorylation modification (see SEQ ID NO: 1), the 5' end of the full-length adaptor p5 sequence has the phosphorylation modification (see SEQ ID NO: 3), and the 5' end of the truncated p5 primer without index has the phosphorylation modification (see SEQ ID NO: 5);
2. When designing the dual index, in addition to the difference of the index sequence itself, the reduction of editing distance caused by the progressive progress of each former and posterior base of the index due to the deletion by synthesis and sequencing errors is also considered, thus the design requirements of meeting the strict 3 editing distances are considered. The P5 end index is considered to be 10 index sequences and the C and A bases before and after them(i.e. C-10 base index-A); The P7 end index is considered to be 10 index sequences and the T and G bases before and after them (T-10 base index-G)
3. Considering the convenience of loading of various models on the double sequencing platform, the present application designs a strict balance on 10 base positions of strict 4 index sequences on the basis of the first two points.

The present application has the following improvements on the basis of Illumina:
P5 truncated amplification primer SEQ ID NO: 1:
/5Phos/AATGATACGGCGACCACCGAGATCTACACNNNNNNNNNNACACTCTTTCCCTA CACGAC, wherein, 10 N(s) represent the index sequence, specifically it can be any sequence in Table 1-1;
P7 truncated amplification primer SEQ ID NO: 2:
CAAGCAGAAGACGGCATACGAGATNNNNNNNNNNGTGACTGGAGTTCAGACGTGT, wherein, 10 N(s) represent the index sequence, specifically it can be any sequence in Table1-2.
P5 full-length adaptor SEQ ID NO: 3:
/5Phos/AATGATACGGCGACCACCGAGATCTACACNNNNNNNNNNACACTCTTTCCCTA CACGACGCTCTTCCGATC*T, wherein, * represents the phosphorthioate modification, 10 N(s) represent the index sequence, specifically it can be any sequence in Table 1-1;
P7 full-length adaptor SEQ ID NO: 4:
/5Phos/GATCGGAAGAGCACACGTCTGAACTCCAGTCACNNNNNNNNNNATCTCGTATG CCGTCTTCTGCTTG, wherein, 10 N(s) represent the index sequence, specifically it can be any sequence in Table 1-2.
P5 phosphorylated amplification primer SEQ ID NO: 5: /5Phos/AATGATACGGCGACCACCGAGAT, the difference is only the phosphorylation from the original Illumina platform;

The P7 amplification primer is the same as the original version of Illumina, which will not be specially indicated here. In addition, there is no difference between the truncated adaptor and the original Illumina's solution.

The following 10 bp 4-balanced index sequences are optimized based on the adaptor sequences of Illumina sequencing platform:

After strict screening of 3 editing distances and the balance of 4 indexes, a total of 412 sequences were screened with a length of 10 bp at the P5 end, the sequences were as shown in Table 1-1; and a total of 432 sequences were screened with a length of 10 bp at the p7 end, the sequences were as shown in Table 1-2.

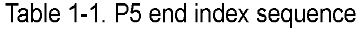

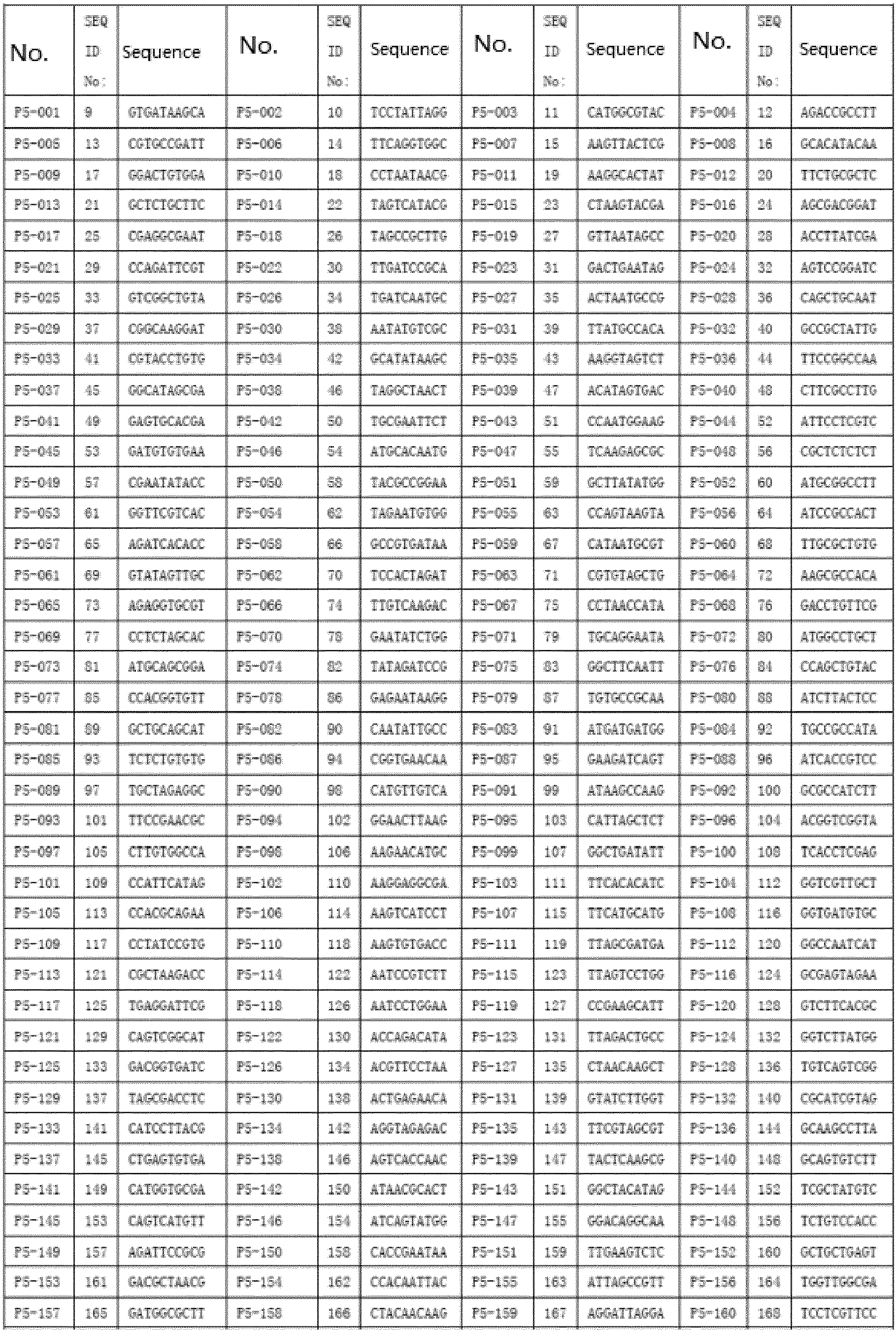

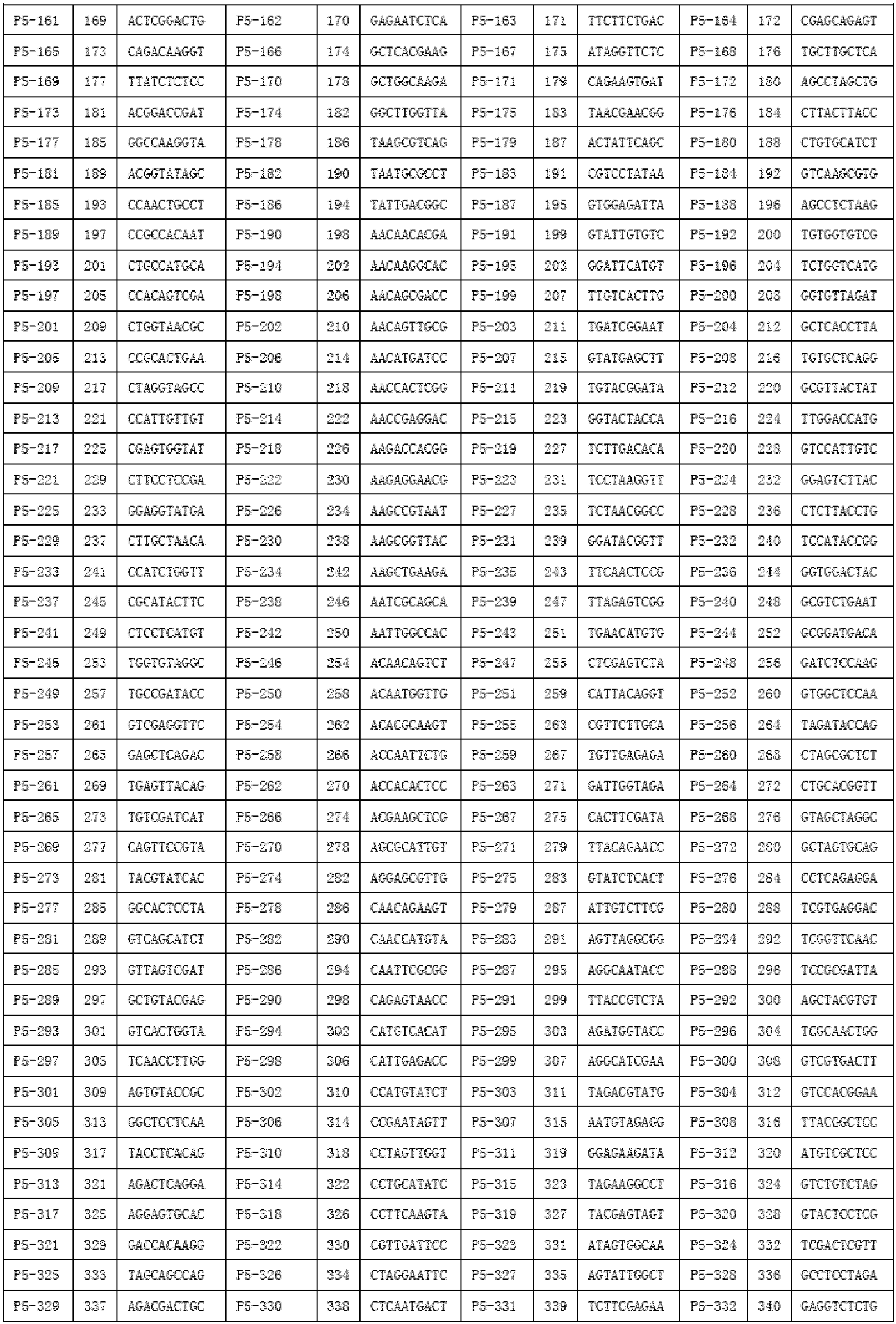

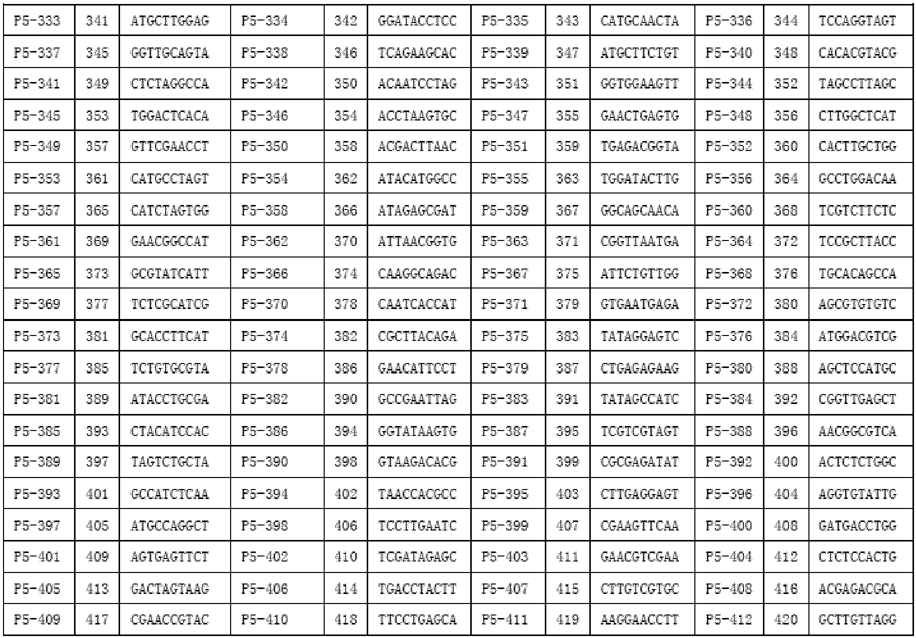

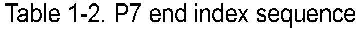

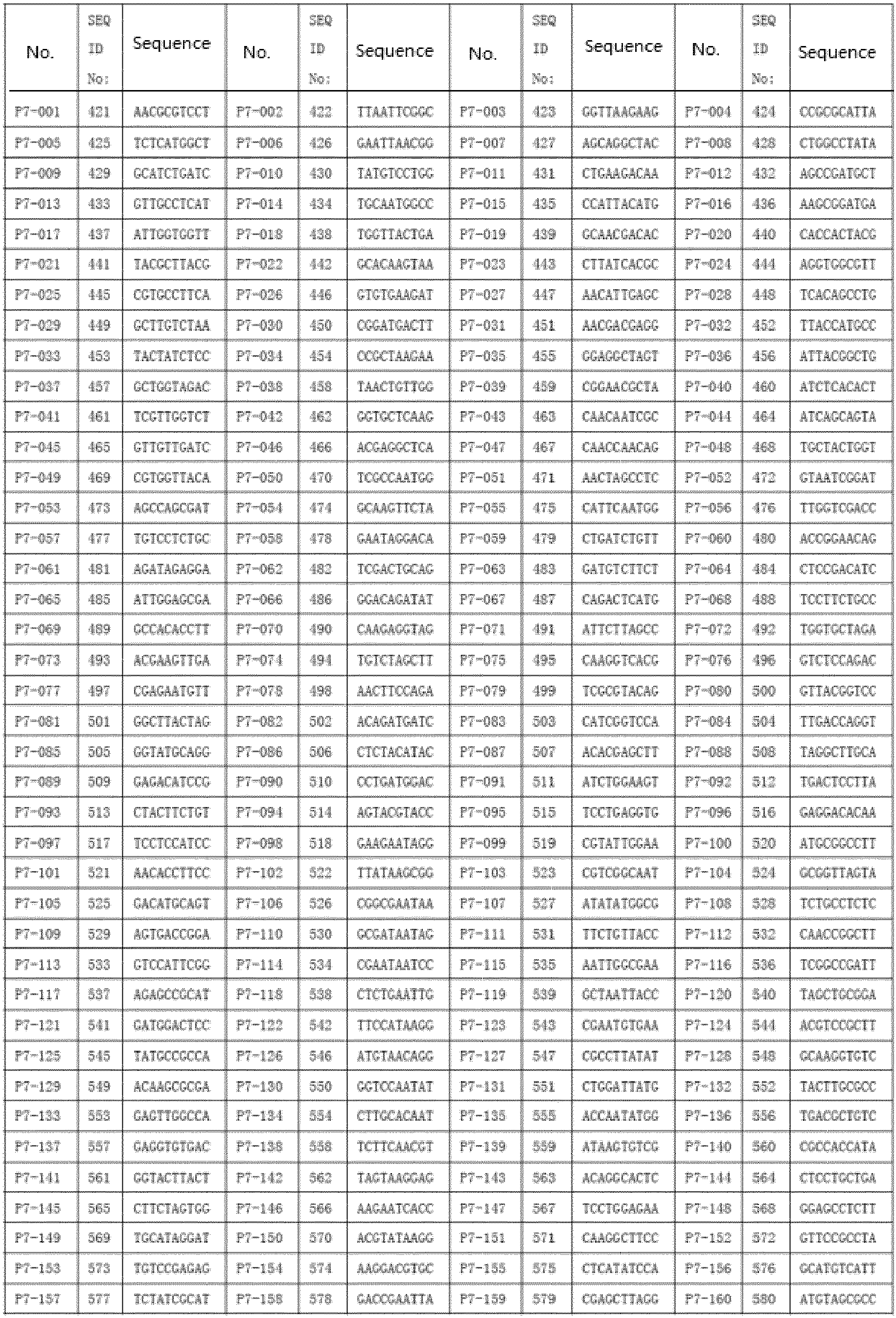

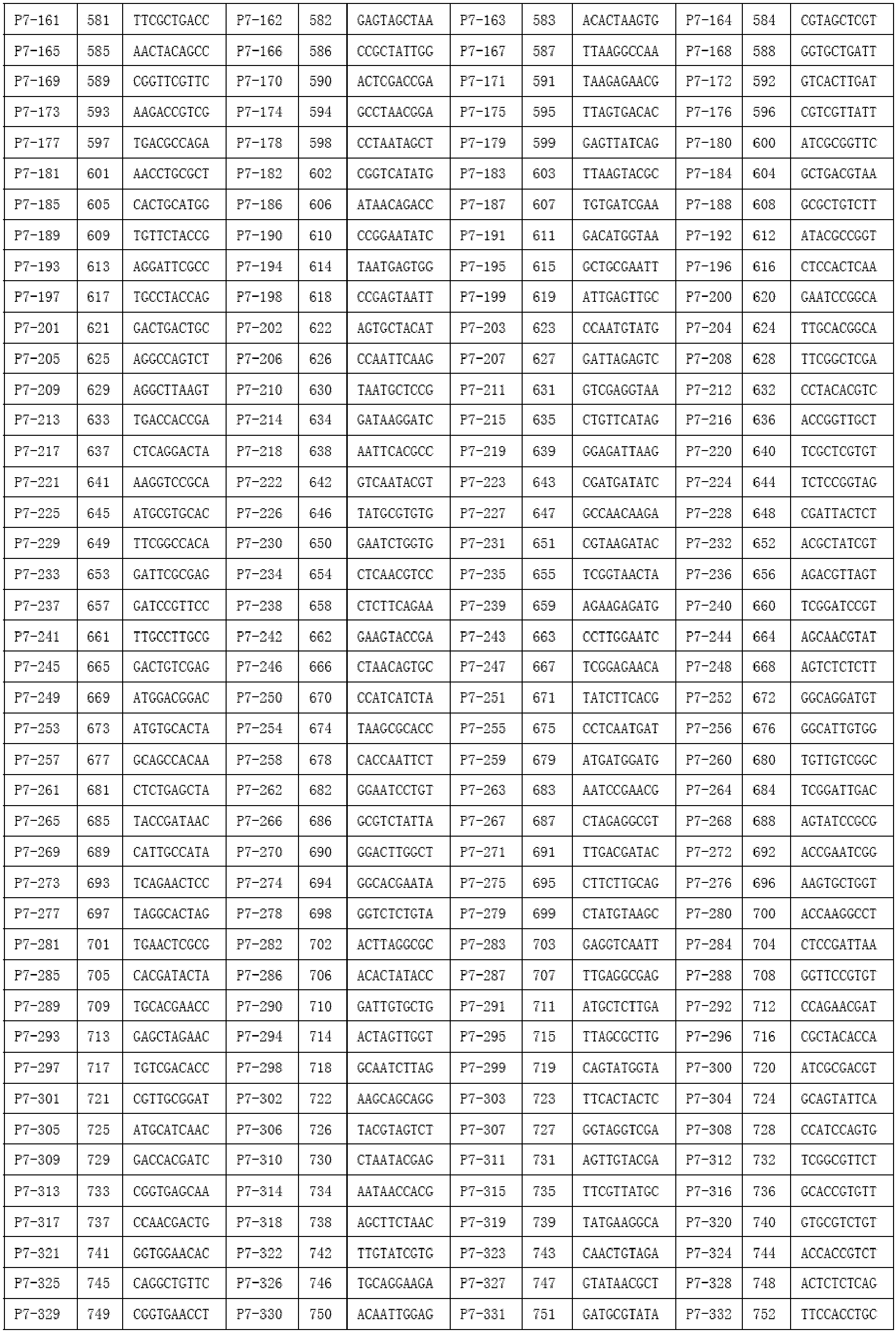

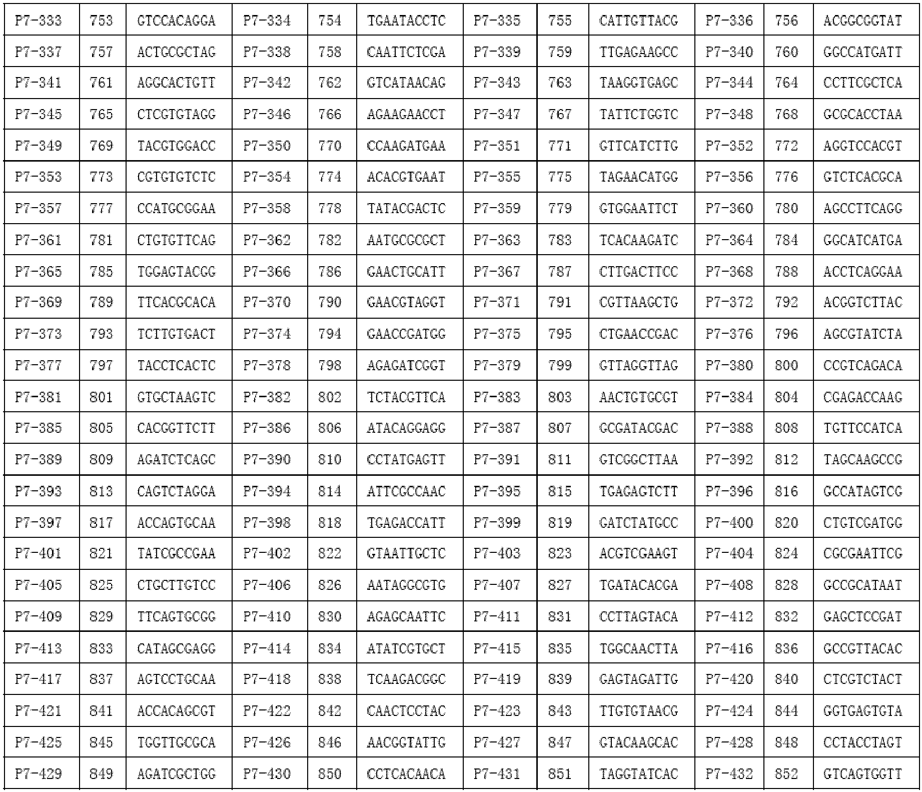

To sum up, this application provides a general library construction solution. This solution can be used for sequencing of all models on both Illumina and MGI platforms. The 4-base balance problem of the index with the smallest unit combination has been considered in the design, and three editing distances can be guaranteed between the forward sequencing and reverse sequencing of strict indexes.

The above improvements have two beneficial effects: first, they can better adapt to Illumina's various models of sequencers, and truly achieve the difference of 3 editing distances between indexes. Second, these improvements can realize direct cyclization and sequencing on the MGI sequencer.

Based on the above research results, the applicant proposes the technical solution protected by the present application. A library construction method compatible with double sequencing platforms is provided, the library construction method comprises: constructing a library for a target sample using primers having a 5' phosphorylation modification or adaptors having a 5' phosphorylation modification to obtain a linear amplification library with a 5' phosphorylation modification, and the linear amplification library with a 5' phosphorylation modification being a linear library suitable for the Illumina sequencing platform; or further cyclizing the linear amplification library with a 5' phosphorylation modification to obtain a cyclization library suitable for the MGI sequencing platform; wherein, the primers having a 5' phosphorylation modification comprises a P5 truncated amplification primer as shown in SEQ ID NO: 1 and a P7 truncated amplification primer as shown in SEQ ID NO: 2; and the adaptors having a 5' phosphorylation modification comprises a P5 full-length adaptor as shown in SEQ ID NO: 3 and a P7 full-length adaptor as shown in SEQ ID NO: 4; wherein, SEQ ID NO: 1: /5Phos/AATGATACGGCGACCACCGAGATCTACACNNNNNNNNNNACACTCTTTCCCTACAC GAC, 10 N(s) represent the P5 end index sequence; SEQ ID NO: 2: CAAGCAGAAGACGGCATACGAGATNNNNNNNNNNGTGACTGGAGTTCAGACGTGT, 10 N(s) represent the P7 end index sequence; SEQ ID NO: 3: /5Phos/AATGATACGGCGACCACCGAGATCTACACNNNNNNNNNNACACTCTTTCCCTACAC GACGCTCTTCCGATC*T, * represents the phosphorthioate modification, 10 N(s) represent the P5 end index sequence; SEQ ID NO: 4: /5Phos/GATCGGAAGAGCACACGTCTGAACTCCAGTCACNNNNNNNNNNATCTCGTATGCCG TCTTCTGCTTG, 10 N(s) represent P7 end index sequence; wherein, each 1 bp upstream and downstream of index sequences comprising the P5 end index sequence or the P7 end index sequence contains at least three editing distances.

In the above improved solution, the linear library constructed by primers or adaptors with 5' phosphorylation modification can be directly sequenced on the Illumina platform. Since the linear library itself has 5' phosphorylation, it can be directly cyclized to prepare a library suitable for the MGI platform. This method is simple for library construction and compatible with double sequencing platform.

In order to further improve the quality of sequencing bases and the accuracy of data splitting during mixed samples sequencing, in one preferred embodiment, the P5 end index sequence is selected from any one of Table 1-1, and the P7 end index sequence is selected from any one of Table 1-2. Since the possible errors or deletions in sequence synthesis have been fully considered in the index sequences in Table 1-1 and Table 1-2, which provides at least 3 editing distances, so the sequencing data of mixed samples can still be correctly split when synthesizing the base deletions.

In another preferred embodiment, there are multiple target samples, the P5 end index sequences corresponding to the multiple target samples are selected from any group of the 4-base balanced index sequences in Table 1-1, and the P7 end index sequences corresponding to the multiple target samples are selected from any group of the 4-base balanced index sequences in Table 1-2, the 4-base balanced index sequences refers to a group of four index sequences balanced, that is, each base of A, T, G and C appears once at each position from position 1 to position 10 of the four index sequences. When multiple target samples are subjected to mix sample sequencing, taking into account the accuracy of the index base reading at the same position (for example, position 3) of different samples, using the preferred index sequences of a group of 4 indexes in Tables 1-1 and 1-2 of this application may keep an equal number of the four base types, achieve the balance of the four base types, thus ensuring the accuracy of the base reading at the same position of each sequence, so as to improve the correct split ratio of the library.

According to whether the 5' phosphorylation modification is in truncated primers or full-length adaptors, the specific workflow of library construction is slightly different. In a preferred embodiment, the primers with a 5' phosphorylation modification is used to perform the library construction on the target sample to obtain the linear amplification library with a 5' phosphorylation modification, comprising: using the truncated adaptors as shown in SEQ ID NO: 7 and SEQ ID NO: 8 to carry out adaptor ligation on the fragments originated from the target sample to obtain the fragments with adaptors; using the primers with a 5' phosphorylation modification as shown in SEQ ID NO: 1 and SEQ ID NO: 2 to amplify the fragments with adaptors to obtain the linear amplification library with 5' phosphorylation modification; wherein, SEQ ID NO: 7: ACACTCTTTCCCTACACGACGCTCTTCCGATC*T, * represents the phosphorthioate modification; SEQ ID NO: 8: /5Phos/GATCGGAAGAGCACACGTCTGAACTCCAGTCAC.

In another preferred embodiment, the adaptors with a 5' phosphorylation modification is used to perform the library construction on the target sample to obtain the linear amplification library with a 5' phosphorylation modification, comprising: using the full-length adaptors as shown in SEQ ID NO: 3 and SEQ ID NO: 4 to carry out adaptor ligation on the fragments originated from the target sample to obtain the libraries with adaptors; using the library amplification primers as shown in SEQ ID NO: 5 and SEQ ID NO: 6 to amplify the libraries with adaptors to obtain the linear amplification library with a 5' phosphorylation modification; wherein, SEQ ID NO: 5 : /5Phos/AATGATACGGCGACCACCGAGAT; SEQ ID NO: 6 : CAAGCAGAAGACGGCATACGA.

The above two ways of linear library construction steps are also applicable to the construction of capture library. That is, after the above steps, the capture library can be obtained further through targeted capturing, and then the library amplification can be performed by library amplification primers with 5' phosphorylation modification to obtain a linear capture library, which is suitable for loading and sequencing on Illumina platform.

For the capture library of MGI platform, the linear amplification library can be subjected to targeted capturing before cyclization. In one preferred embodiment, the captured library after the targeted capturing is amplified using library amplification primers with a 5' phosphorylation modification to obtain a linear amplification captured library, the linear amplification captured library is cyclized to obtain the cyclization library suitable for the MGI sequencing platform; preferably, the library amplification primers with a 5' phosphorylation modification comprises a P5 phosphorylation primer as shown in SEQ ID NO: 5 and a P7 primer as shown in SEQ ID NO: 6.

According to a second aspect of the present application, a library construction kit compatible with double sequencing platforms is provided, the library construction kit comprises any of the following combinations: 1) combination 1: a P5 truncated amplification primer as shown in SEQ ID NO: 1 and a P7 truncated amplification primer as shown in SEQ ID NO: 2, wherein, SEQ ID NO: 1: /5Phos/AATGATACGGCGACCACCGAGATCTACACNNNNNNNNNNACACTCTTTCCCTACAC GAC, 10 N(s) represent the P5 end index sequence; SEQ ID NO: 2: CAAGCAGAAGACGGCATACGAGATNNNNNNNNNNGTGACTGGAGTTCAGACGTGT, 10 N(s) represent the P7 end index sequence; 2) combination 2: P5 full-length adaptor SEQ ID NO: 3 and P7 full-length adaptor SEQ ID NO: 4, wherein, SEQ ID NO: 3: /5Phos/AATGATACGGCGACCACCGAGATCTACACNNNNNNNNNNACACTCTTTCCCTACAC GACGCTCTTCCGATC*T, * represents the phosphorthioate modification, 10 N(s) represent the P5 end index sequence; SEQ ID NO: 4: /5Phos/GATCGGAAGAGCACACGTCTGAACTCCAGTCACNNNNNNNNNNATCTCGTATGCCG TCTTCTGCTTG, 10 N(s) represent the P7 end index sequence; wherein, each 1 bp sequence upstream and downstream of index sequences comprising the P5 end index sequence or the P7 end index sequence contains at least three editing distances.

Further, the P5 end index sequence is selected from any one of Table 1-1, and the P7 end index sequence is selected from any one of Table 1-2.

Further, the library construction kit comprises 412 P5 end index sequences and 432 P7 end index sequences, the P5 end index sequences are as shown in Table 1-1, and the P7 end index sequences are as shown in Table 1-2, wherein, the P5 end index sequence and/or the P7 end index sequence are used in a coordination way of a group of 4-base balanced tag sequences.

According to a third aspect of the present application, a library construction element compatible with double sequencing platforms is provided, the library construction element is selected from any of the following combinations: 1) combination 1: a P5 truncated amplification primer as shown in SEQ ID NO: 1 and a P7 truncated amplification primer as shown in SEQ ID NO: 2, wherein, SEQ ID NO: 1: /5Phos/AATGATACGGCGACCACCGAGATCTACACNNNNNNNNNNACACTCTTTCCCTACAC GAC, 10 N(s) represent the P5 end index sequence; SEQ ID NO: 2: CAAGCAGAAGACGGCATACGAGATNNNNNNNNNNGTGACTGGAGTTCAGACGTGT, 10 N(s) represent P7 end index sequence; 2) combination 2: a P5 full-length adaptor shown in SEQ ID NO: 3 and a P7 full-length adaptor as shown in SEQ ID NO: 4, wherein, SEQ ID NO: 3: /5Phos/AATGATACGGCGACCACCGAGATCTACACNNNNNNNNNNACACTCTTTCCCTACAC GACGCTCTTCCGATC*T, * represents the phosphorthioate modification, 10 N(s) represent the P5 end index sequence; SEQ ID NO: 4: /5Phos/GATCGGAAGAGCACACGTCTGAACTCCAGTCACNNNNNNNNNNATCTCGTATGCCG TCTTCTGCTTG, 10 N(s) represent the P7 end index sequence; wherein, each 1 bp sequence upstream and downstream of index sequences comprising the P5 end index sequence or the P7 end index sequence contains at least three editing distances.

Further, the P5 end index sequence is selected from any one of Table 1-1, and the P7 end index sequence is selected from any one of Table 1-2.

Further, the library construction element is an amplification primer composition or an adaptor composition, the amplification primer composition comprises a combination of multiple groups of P5 truncated amplification primers and/or multiple groups of P7 truncated amplification primers, each group of the P5 truncated amplification primers comprises a 4-base balanced index sequences selected from any group of Table 1-1, and each group of the P7 truncated amplification primers comprises a 4-base balanced index sequences selected from any group of Table 1-2; the adaptor composition comprises multiple groups of P5 full-length adaptors and/or multiple groups of P7 full-length adaptors, each group of the P5 full-length adaptors comprises a 4-base balanced tag sequence selected from any group of Table 1-1, and each group of the P7 full-length adaptors comprises a 4-base balanced index sequences selected from any group of Table 1-2; the 4-base balanced index sequences refers to a group of four index sequences balanced, that is, each base of A T, G and C appear once at each position from position 1 to position 10 of the four index sequences.

Advantageous effects of the present application will be further described below in combination with specific examples.

It should be noted that the following embodiments were carried out using NadPrep^{™} DNA library construction workflow provided by Nanodigmbio (Nanjing) Co., Ltd. Instruction V3.4 with NadPrep^{™} Library construction kit for Illumina. The specific workflow is briefly described as follows:
Fragmentation of DNA sample - end repair and addition of A - adaptor ligation - fragments screening - PCR amplification - library purification, quantification and quality inspection - sequencing on Illumina/MGI platform or sequencing after targeted library capture.

It should also be noted that the following embodiments are only illustrative and do not limit the method of the present application to the following methods.

### Example 1: Comparison between the library construction solutions I and II of the present application and the library construction solution by the separate Illuminia platform in the prior art

### Procedures:

The library construction procedures can make reference to NadPrep^{™} DNA Library construction kit for Illumina (202105Version3.4) instruction, the only difference lies in the difference between the adaptors and the amplification primers, specifically as follows:

### (1) Solution I of the invention:

Truncated adaptor sequence (Truncated adaptor sequence was consistent with the adaptor sequence sequenced by single Illumina platform, both of which are SEQ ID NO: 7 and SEQ ID NO: 8 as below):
ACACTCTTTCCCTACACGACGCTCTTCCGATC*T, * represents the phosphorthioate modification, (SEQ ID NO: 7)
/5Phos/GATCGGAAGAGCACACGTCTGAACTCCAGTCAC (SEQ ID NO: 8)

The truncated amplification primers are the above SEQ ID NO: 1 and SEQ ID NO: 2, wherein the index sequence of SEQ ID NO:1 is selected from P5-001 to P5-096 in Table 1-1, and the index sequence of SEQ ID NO:2 is selected from P7-001 to P7-096 in Table 1-2.

The characteristics of solution I are as follows:
1) the truncated P5 primer was subjected to phosphorylation modification to be compatible with the MGI sequencing platform.
2) the indexes in the intermediate were optimized to consider the possible base progression of deletion by synthesis and 3 editing distances compatible with platform factors.
3) the absolute balance of 10 base positions of 4 indexes was benefit to the schedule of loading on the two-color and four-color sequencing platforms.

### (2) Solution II of the present application:

the full-length adaptor sequences: SEQ ID NO: 3+SEQ ID NO: 4, wherein the index sequence of SEQ ID NO: 4 is selected from P5-001 to P5-096 in Table 1-1, and the index sequence of SEQ ID NO: 4 is selected from P7-001 to P7-096 in Table 1-2.
P5 phosphorylated amplification primer is shown in SEQ ID NO: 5: /5Phos/AATGATACGGCGACCACCGAGAT; and
P7 primer is shown in SEQ ID NO: 6: CAAGCAGAAGACGGCATACGA.

The characteristics of solution II are as follows:
1) The 5' end of the full-length P5 adaptor was subjected to phosphorylation modification, and the P5 end of the subsequent amplification primer was subjected to 5' phosphorylation modification, so as to be compatible with the MGI sequencing platform;
2) Full-length library construction can be created by PCR-free;
3) the index in the intermediate was optimized to consider the possible base progression of deletion by synthesis and 3 editing distances compatible with platform factors.
4) the absolute balance of 10 base positions of 4 indexes was conducive to the arrangement of computer operation on the two-color and four-color sequencing platforms.

### (3) Control solution

The control solution is to use the products currently launched by Nanodigmbio on the Illumina platform cooperated with 384 UDI adaptors of IDT for library construction, and use common P5 and P7 primers for amplification. Specific procedures can make reference to NadPrep^{™} DNA Library construction kit for Illumina (202105Version3.4) instruction.

**Table 2: Comparison of Output of Three Library Construction Solutions**

| Solutions | DNA input | Number of amplification cycles |
|---|---|---|
| Solution I | 50 ng | 6 |
| Solution II | 50 ng | 7 |
| Control | 50 ng | 7 |

The library construction outputs of the libraries sequenced on the Illumina platform in Solution I and Solution II of the present application and control are shown in Figure 12. Solution I of the present invention requires only 6 cycles at the same input of 50ng to achieve the output of 7 cycles in Solution II and control. In specific applications, the truncated Solution I has more advantages in library construction output and compatibility. Compatibility here refers to the compatibility of universal truncated adaptor, molecular index adaptor used in plasma, truncated methylated molecular index adaptor and library construction of amplicon. Solution II of the present application has the advantage that it can be used for PCR free library construction, and the output of Solution II is equivalent to that of the library produced by the control library construction method.

### Example 2 The same library may be sequenced on double platforms

### Procedures:

The library construction procedures can make reference to NadPrep^{™} DNA Library construction kit for Illumina (202105Version3.4) instruction, the adaptor solution of the library construction solution was carried out according to Solution I of the present application, and the library construction was started from 100 ng DNA standard interrupted by ultrasound (Promage Company). The truncated adaptor in Solution I of the present application and the amplification primers of SEQ ID NO: 1 and SEQ ID NO: 2 were used for amplification. The first 96 in Table 1-1 of the P5 end index No. and the first 96 in Table 1-2 of the P7 end index No. were combined correspondingly, for example, the combination can be a combination of P5-001 and P7-001, and a combination of P5-002 and P7-002, and so on, until a combination of P5-096 and P7-096. However, it should be noted that the combinations here was not the only limited combination mode. Any combination of a group of four indexes can be used for mixed samples sequencing (for example, it can be a combination of any of P5 and P7 in P5-001 to P5-004 and P7-001 to P7-004, and the other four, for example a combination of any of P5 and P7 in P5-097 to P5-100 and P7-097 to P7-100, and so on).

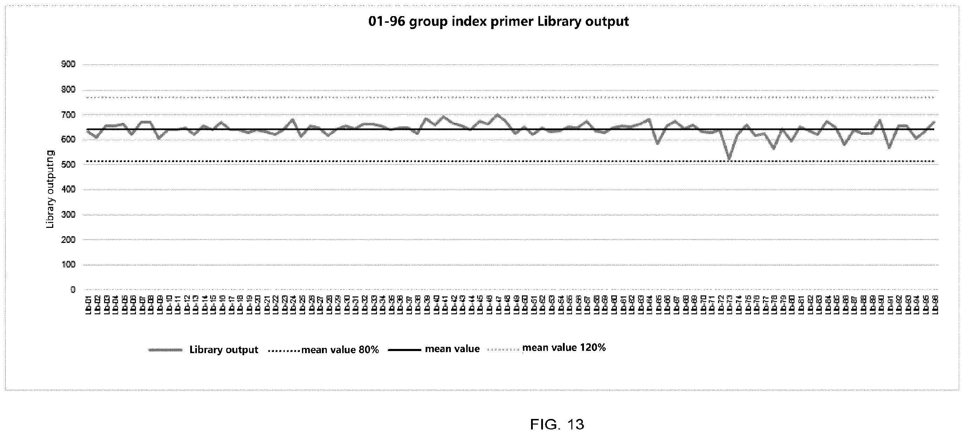

The number of amplification cycles was 5, and the library output was shown in Figure 13. All library outputs were between 80% and 120% above or below the mean value, indicating that the amplification efficiency of Solution I of the present application was relatively balanced.

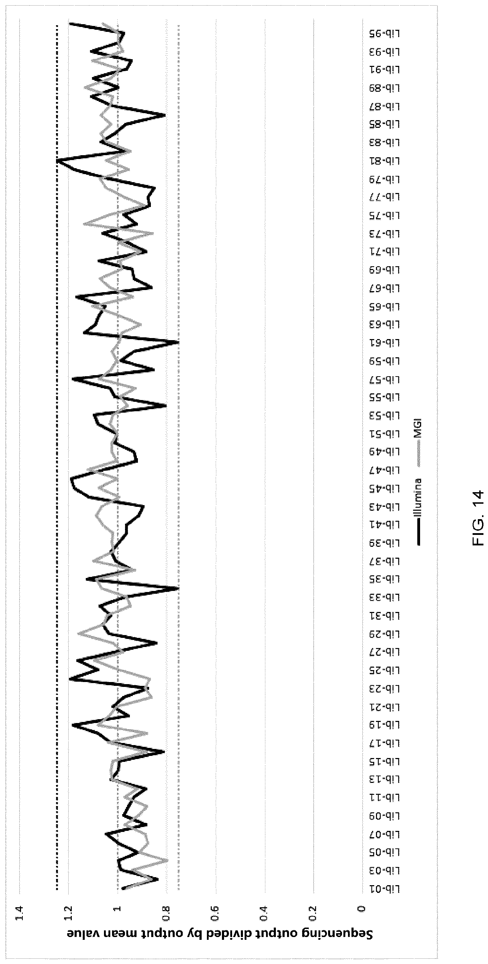

At the same time, the libraries constructed by these 96 groups of primers were sequenced on the Illumina and MGI sequencing platforms for whole genome sequencing (WGS) mixed in equal proportions. The sequenced data were split, and the split data were homogenized. The sequencing data of each library was divided by the average value of all data. The final results are shown in Figure 14. The output was between 75% and 125%, indicating that the Solutions of the present application has a consistent performance on both platforms. A set of compatible library construction solutions can be used to solve the problem of sequencing on both platforms.

### Example 3 The significance of four groups of index balance in scheduling and effective splitting

### Procedures:

The library construction procedures can make reference to NadPrep^{™} DNA Library construction kit for Illumina (202105Version3.4) instruction, the 8 groups and 12 groups of primers of the present application were used for library construction and sequencing, respectively, and the 8 groups and 12 groups of Illumina platform officially recommended IDT version library construction were used for analysis on the Hiseq X Ten separate package chip channel (lane).

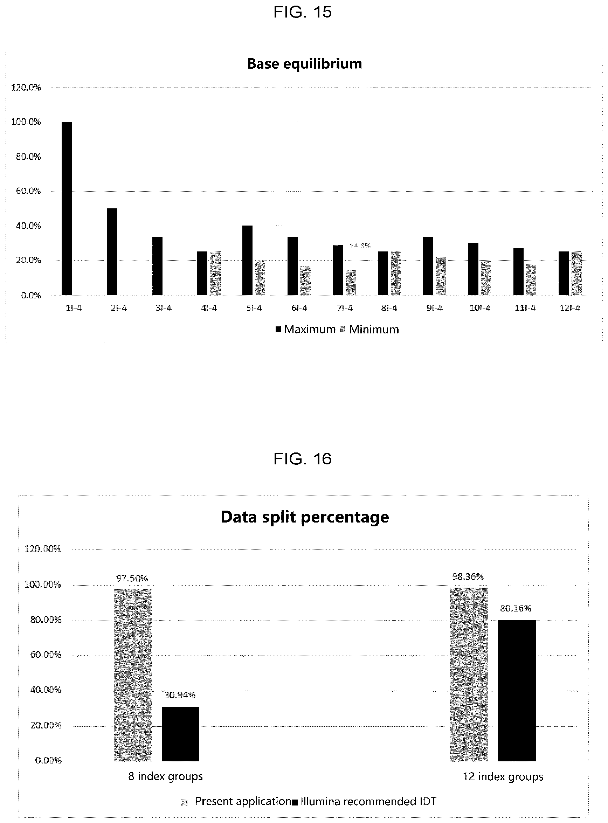

As shown in Figure 15, the highest and lowest base proportions of 1-12 groups balanced by four groups of indexes of the present application when scheduling and sequencing. In the combination of greater than 4 or more in the four groups of index balances of the present application, the lowest value was 14.3%, which was greater than the minimum requirement of 12.5% specified by MGI. In the first 8 groups and the first 12 groups of IDT version, the lowest values were 0 and 8.3%, as shown in Figure 11.

As shown in Figure 16, the final splitting result of the present application shows that because the bases of 8 groups and 12 groups at each position are balanced, the data splitting percentage can reach more than 97%. the data splitting of 8 groups and 12 groups of IDT version recommended by Illumina are not ideal, which are more than 30% and 80%, respectively. Since Hiseq X Ten is a four-color fluorescence channel sequencer, the base imbalance seriously affects the sequencing quality and effective data splitting.

From the above description, it can be seen that the above embodiments of the present application achieves the following technical effects: the present application has designed and optimized a compatible library construction method and a library construction method after hybrid capture. The library construction and hybrid capture method can realize sequencing on the Illumina platform, or sequencing on the MGI sequencing platform after the library is directly cycled. In the design of the index, the first base upstream and downstream the index is also fully considered to ensure that three editing distances can be guaranteed during deletion and insertion, so that the data will not be split wrongly. At the same time, the strict four balance design avoids the difficulty of scheduling during sequencing, which is benefit to ensuring the sequencing quality and effective data splitting.

The above description is only the preferred examples of the present application and is not intended to limit. For those skilled in the art, various modifications and changes can be made to the present invention. Any modifications, equivalent substitutions, improvements, and the like made within the spirit and principle of the present invention shall be included into the scope of protection of the present invention.

## Claims

1. A library construction method compatible with double sequencing platforms, wherein the library construction method comprises:
constructing a library for a target sample with primers having a 5' phosphorylation modification or adaptors having a 5' phosphorylation modification to obtain a linear amplification library with a 5' phosphorylation modification, and the linear amplification library with a 5' phosphorylation modification being a linear library suitable for the Illumina sequencing platform; or
constructing a library a target sample with primers having a 5' phosphorylation modification or adaptors having a 5' phosphorylation modification to obtain a linear amplification library with a 5' phosphorylation modification, and cyclizing the linear amplification library with a 5' phosphorylation modification to obtain a cyclization library suitable for the MGI sequencing platform;
wherein, the primers having a 5' phosphorylation modification comprises a P5 truncated amplification primer as shown in SEQ ID NO: 1 and a P7 truncated amplification primer as shown in SEQ ID NO: 2; and the adaptors having a 5' phosphorylation modification comprises a P5 full-length adaptor as shown in SEQ ID NO: 3 and a P7 full-length adaptor as shown in SEQ ID NO: 4;
wherein, SEQ ID NO: 1:
/5Phos/AATGATACGGCGACCACCGAGATCTACACNNNNNNNNNNACACTCTTTCCCTACAC GAC, 10 N(s) represent a P5 end index sequence;
SEQ ID No: 2:
CAAGCAGAAGACGGCATACGAGATNNNNNNNNNNGTGACTGGAGTTCAGACGTGT, 10 N(s) represent a P7 end index sequence;
SEQ ID No: 3:
/5Phos/AATGATACGGCGACCACCGAGATCTACACNNNNNNNNNNACACTCTTTCCCTACAC GACGCTCTTCCGATC*T, 10 N(s) represent a P5 end index sequence, and * represents a phosphorthioate modification;
SEQ ID No: 4:
/5Phos/GATCGGAAGAGCACACGTCTGAACTCCAGTCACNNNNNNNNNNATCTCGTATGCCG TCTTCTGCTTG, 10 N(s) represent a P7 end index sequence;
wherein, each 1 bp upstream and downstream of index sequences comprising the P5 end index sequence or the P7 end index sequence comprises at least three editing distances.

2. The library construction method according to claim 1, wherein, the P5 end index sequence is selected from any one of Table 1-1, and the P7 end index sequence is selected from any one of Table 1-2.

3. The library construction method according to claim 2, wherein, there are multiple target samples, multiple P5 end index sequences corresponding to the multiple target samples are selected from any group of the 4-base balanced index sequences in Table 1-1, and multiple P7 end index sequences corresponding to the multiple target samples are selected from any group of the 4-base balanced index sequences in Table 1-2, wherein the 4-base balanced index sequence refers to a group of four index sequences balanced, that is, each base of A, T, G and C appears once at each position from position 1 to position 10 of the four index sequences.

4. The library construction method according to claim 3, wherein, constructing a library for a target sample using primers with a 5' phosphorylation modification and to obtain the linear amplification library with a 5'phosphorylation modification, comprising:
ligating truncated adaptors as shown in SEQ ID NO: 7 and SEQ ID NO: 8 into fragments originated from the target samples to obtain fragments with adaptors; and
amplifying the fragments with adaptors with the primers having a 5' phosphorylation modification as shown in SEQ ID NO: 1 and SEQ ID NO: 2 to obtain the linear amplification library with the 5' phosphorylation modification;
wherein, SEQ ID NO: 7: ACACTCTTTCCCTACACGACGCTCTTCCGATC*T, * represents a phosphorthioate modification;
SEQ ID NO: 8: /5Phos/GATCGGAAGAGCACACGTCTGAACTCCAGTCAC.

5. The library construction method according to claim 3, wherein, constructing a library for a target sample with adaptors having a 5' phosphorylation modification to obtain the linear amplification library with a 5'phosphorylation modification, comprising:
ligating the full-length adaptor as shown in SEQ ID NO: 3 and SEQ ID NO: 4 into fragments originated from the target sample to obtain the libraries with adaptors;
amplifying the libraries with adaptors with library amplification primers as shown in SEQ ID NO: 5 and SEQ ID NO: 6 to obtain the linear amplification library with a 5'phosphorylation modification;
wherein, SEQ ID NO: 5 : /5Phos/AATGATACGGCGACCACCGAGAT;
SEQ ID NO: 6 : CAAGCAGAAGACGGCATACGA.

6. The library construction method according to any one of claims 1 to 5, wherein, before cyclizing the linear amplification library, the library construction method further comprises a step of targeted capturing the linear amplification library;
preferably, amplifying the captured library after the targeted capturing with library amplification primers having a 5'phosphorylation modification to obtain a linear amplification captured library,
cyclizing the linear amplification captured library to obtain the cyclization library suitable for the MGI sequencing platform;
preferably, the library amplification primers having a 5'phosphorylation modification comprise a P5 phosphorylation primer as shown in SEQ ID NO: 5 and a P7 primer as shown in SEQ ID NO: 6.

7. A library construction kit compatible with double sequencing platforms, wherein, the library construction kit comprises any of the following combinations:
1) combination 1: a P5 truncated amplification primer as shown in SEQ ID NO: 1 and a P7 truncated amplification primer as shown in SEQ ID NO: 2, wherein, SEQ ID NO: 1:
/5Phos/AATGATACGGCGACCACCGAGATCTACACNNNNNNNNNNACACTCTTTCCCTACAC GAC, 10 N(s) represent a P5 end index sequence;
SEQ ID NO: 2
CAAGCAGAAGACGGCATACGAGATNNNNNNNNNNGTGACTGGAGTTCAGACGTGT, 10 N(s) represent a P7 end index sequence;
2) combination 2: a P5 full-length adaptor as shown in SEQ ID NO: 3 and a P7 full-length adaptor as shown in SEQ ID NO: 4, wherein,
SEQ ID NO: 3:
/5Phos/AATGATACGGCGACCACCGAGATCTACACNNNNNNNNNNACACTCTTTCCCTAC ACGACGCTCTTCCGATC*T, 10 N(s) represent a P5 end index sequence, and * represents a phosphorthioate modification;
SEQ ID NO: 4:
/5Phos/GATCGGAAGAGCACACGTCTGAACTCCAGTCACNNNNNNNNNNATCTCGTATGCCG TCTTCTGCTTG, 10 N(s) represent a P7 end index sequence;
wherein, each 1 bp upstream and downstream of index sequences comprising the P5 end index sequence or the P7 end index sequence comprises at least three editing distances.

8. The library construction kit according to claim 7, wherein, the P5 end index sequence is selected from any one of Table 1-1, and the P7 end index sequence is selected from any one of Table 1-2.

9. The library construction kit according to claim 7, wherein, the library construction kit comprises 412 P5 end index sequences and 432 P7 end index sequences, the P5 end index sequences are as shown in Table 1-1, and the P7 end index sequences are as shown in Table 1-2,
wherein, the P5 end index sequence and/or the P7 end index sequence are used in a coordination way of a group of 4-base balanced index sequences.

10. The library construction kit according to any one of claims 7 to 9, wherein, the library construction kit further comprises library amplification primers as shown in SEQ ID NO: 5 and SEQ ID NO: 6, and/or truncated adaptors as shown in SEQ ID NO: 7 and 8.

11. A library construction element compatible with double sequencing platforms, wherein, the library construction element is selected from any of the following combinations:
1) combination 1: a P5 truncated amplification primer as shown in SEQ ID NO: 1 and a P7 truncated amplification primer as shown in SEQ ID NO: 2, wherein, SEQ ID NO: 1:
/5Phos/AATGATACGGCGACCACCGAGATCTACACNNNNNNNNNNACACTCTTTCCCTACAC GAC, 10 N(s) represent a P5 end index sequence;
SEQ ID NO: 2
CAAGCAGAAGACGGCATACGAGATNNNNNNNNNNGTGACTGGAGTTCAGACGTGT, 10 N(s) represent a P7 end index sequence;
2) combination 2: a P5 full-length adaptor as shown in SEQ ID NO: 3 and a P7 full-length adaptor as shown in SEQ ID NO: 4, wherein,
SEQ ID NO: 3:
/5Phos/AATGATACGGCGACCACCGAGATCTACACNNNNNNNNNNACACTCTTTCCCTACAC GACGCTCTTCCGATC*T, 10 N(s) represent a P5 end index sequence, and * represents a phosphorthioate modification,
SEQ ID NO: 4:
/5Phos/GATCGGAAGAGCACACGTCTGAACTCCAGTCACNNNNNNNNNNATCTCGTATGCCG TCTTCTGCTTG, 10 N(s) represent a P7 end index sequence;
wherein, each 1 bp upstream and downstream of index sequences comprising the P5 end index sequence or the P7 end index sequence contains at least three editing distances.

12. The library construction element according to claim 11, wherein, the P5 end index sequence is selected from any one of Table 1-1, and the P7 end index sequence is selected from any one of Table 1-2.

13. The library construction element according to claim 11, wherein, the library construction element is an amplification primer composition or an adaptor composition,
the amplification primer composition comprises a combination of multiple groups of P5 truncated amplification primers and/or multiple groups of P7 truncated amplification primers, each group of the P5 truncated amplification primers comprises a 4-base balanced index sequences selected from any group of Table 1-1, and each group of the P7 truncated amplification primers comprises a 4-base balanced index sequences selected from any group of Table 1-2;
the adaptor composition comprises multiple groups of P5 full-length adaptors and/or multiple groups of P7 full-length adaptors, each group of the P5 full-length adaptors comprises a 4-base balanced tag sequences selected from any group of Table 1-1, and each group of the P7 full-length adaptors comprises a 4-base balanced index sequences selected from any group of Table 1-2;
the 4-base balanced index sequences refer to a group of four index sequences balanced, that is, each base of A T, G and C appear once at each position from position 1 to position 10 of the four index sequences.
